# EUROPEAN PATENT SPECIFICATION

(11) **EP 0 290 644 B1**
(45) Date of publication and mention of the grant of the patent: **15.11.1995**
(21) Application number: 87106858.1
(22) Date of filing: 12.05.1987
(51) Int. Cl.: A61K 38/24

(54) **Subcutaneous administration of human chorionic gonadotrophin**
Subkutane Verabreichung des humanen Choriongonadotropins
Administration sous-cutanée de gonadotrophine chorionique humaine

(43) Date of publication of application: 17.11.1988
(73) Proprietor: Serono Pharmazeutische Präparate GmbH, D-79100 Freiburg (DE)
(72) Inventor: Saal, Werner, Dr. med., D-6501 Budenheim (DE); Happ, Joachim, Prof. Dr. med., D-6000 Frankfurt/M (DE)
(74) Representative: Kraus, Walter, Dr.

(56) References cited:
- WO-A-86/04241
- FR-M- 2 608
- GB-A- 2 174 600

## Description

The present invention relates to the administration of Human Chorionic Gonadotrophin (HCG) by the subcutaneous route in the treatment of male sexual disorders or infertility.

It is generally known that HCG is a hormone derived from the placenta which exhibits LH-like effects and is responsible for the maintenance of the corpus luteum and the stimulation of progesterone production.

HCG has been clinically used for a number of years for the treatment of female and male infertility statuses and sexual disorders. So far, adminstration of HCG has been carried out exclusively by intramuscular (i.m.) injection.

FR-M-2 608 relates to long acting preparations of gonadotrophines obtained from urine.

As i.m. injections are generally performed by the physician or by the medical professional staff, the patient is expected to visit a surgery or a hospital regularly in order to receive treatment. Besides the discomfort created, the time taken up by this type of application often leads to unsatisfactory compliance by the patient, particularly when the treatment extends over several months.

It has now been found that Human Chorionic Gonadotrophin can be efficaciously administered by subcutaneous injection, thus rendering possible the self-administration by the patient and consequently improving patients' cooperation and compliance. These advantages are even more evident in the case of a long-term therapy, such as the one applied to the treatment of male sexual disorders. Another advantage of the s.c. administration lies in a substantially lower complication rate due to possible side effects, such as abscess formation and nerve lesions. Additional advantages will become evident from the description which follows.

The subcutaneous administration of Human Menopausal Gonadotrophin (HMG) has already been described (Nakamura Y. et al., Fertility and Sterility, 46(1): 46-54, 1986) in connection with the treatment of female infertility by pulsatile administration of HMG via the subcutaneous route using a portable peristaltic pump. Advantages were shown over the pulsatile administration performed via the intravenous route as well as over the conventional i.m. administration.

However, data concerning the pharmacokinetics and pharmacodynamics as well as the biological effects of HCG following s.c. injection were not available prior to the present invention which is based on a study designed so as to compare the pharmacokinetics of HCG following intramuscular and subcutaneous administrations.

The results show that the subcutaneous administration of HCG displays the same biological effects as the intramuscular administration at the same dose level. This is surprising because a higher dose requirement of subcutaneously administered HCG with respect to the intramuscular route would have been expected in order to obtain the same end result.

In fact, as will appear more clearly from the detailed description which follows, the expected reduced resorption of HCG when administered by the s.c. route is compensated by its extended half life. However, despite this different pharmacokinetic behaviour of HCG between the two administration routes, the desired end result, i.e., the Testosterone serum level, is practically identical in both cases. Identical results are also obtained as far as the decrease of Follicle Stimulating Hormone (FSH) and Luteinizing Hormone (LH) serum levels are concerned.

An additional advantage that can be drawn from the result of the study carried out in accordance with this invention is that the slow absorption avoids excessively high HCG peaks while stimulating Testosterone serum levels which are identical to those obtained by administering HCG intramuscularly.

The following detailed description further illustrates the invention:

### Pharmacokinetic study

a) Patient population
   24 male volunteers aged between 18 and 41 years participated in the study. All patients were endocrinologically and andrologically inconspicuous. Patients with liver and kidney dysfunctions, as well as arterial hypertension, were not admitted to the study.
b) Medication
   One ampoule of Pregnesin^{(R)} 5000 IU (Serono, Freiburg, FRG), corresponding to 5000 IU HCG dissolved in 1 ml of solvent, was administered.
c) Test procedure
   The population was subdivided into two randomized groups of 12 test persons each. Group 1 one was given 5000 IU of HCG i.m. (intragluteally). Group 2 was given the same dosage s.c. on the ventral side of the thigh. The substance was in each case administered at 8 a.m.
   Blood samples for the determination of HCG, LH and testosterone were taken from a cubital vein at the following times: immediately prior to the administration of HCG and 1, 2, 4, 6, 8, 12, 16, 22, 26, 30, 36, 48, 72, 96, 120 and 144 hours after the injection. The serum level of FSH was determined at the following times: prior to the injection and after 4, 12, 22, 48, 72, 96, 120 and 144 hours. Immediately after collection the samples were centrifuged and the sera stored at -20°C until performance of the analyses.
d) Laboratory methods
   Due to their high sensitivity and negligible cross-reactivity to related hormones, the RIA-kits HCG, LH and FSH MAIA-clone^{(R)} as well as testosterone MAIA^{(R)} (Serono Diagnostika Freiburg, FRG) were used as test systems.
   The tests were conducted in accordance with the user information included in each test kit. All tests were performed in duplicates. tests. The resulting mean values were considered in the subsequent assessment.

Results are shown by the following self-explanatory Tables 1 to 4.

**Table 1**

| HCG serum concentrations (mU/ml) following i.m and s.c. injection of 5.000 IU HCG | | |
|---|---|---|
| hours after injection | i.m. | s.c. |
| 0 | 0.41 ± 0.28 | 0.26 ± 0.27 |
| 1 | 107.80 ± 51.82 | 14.44 ± 8.65 |
| 2 | 252.17 ± 55.03 | 42.95 ± 15.99 |
| 4 | 382.44 ± 64.06 | 96.86 ± 30.45 |
| 6 | 406.97 ± 60.19 | 120.82 ± 38.79 |
| 8 | 404.08 ± 43.83 | 145.83 ± 43.42 |
| 12 | 387.75 ± 66.05 | 179.76 ± 49.47 |
| 16 | 345.73 ± 55.36 | 187.47 ± 55.90 |
| 22 | 285.66 ± 56.89 | 163.96 ± 43.94 |
| 26 | 284.14 ± 47.96 | 174.35 ± 41.39 |
| 30 | 255.11 ± 44.31 | 169.00 ± 44.83 |
| 36 | 217.52 ± 36.56 | 160.81 ± 44.43 |
| 48 | 166.32 ± 28.62 | 122.27 ± 33.78 |
| 72 | 87.61 ± 20.07 | 74.23 ± 19.70 |
| 96 | 53.21 ± 12.85 | 49.26 ± 12.75 |
| 120 | 32.64 ± 9.78 | 31.75 ± 9.39 |
| 144 | 21.17 ± 6.53 | 20.57 ± 6.15 |

**Table 2**

| Testosterone serum level (ng/ml) following i.m and s.c. injection of 5.000 IU HCG | | |
|---|---|---|
| hours after injection | i.m. | s.c. |
| 0 | 7.94 ± 2.31 | 7.74 ± 2.70 |
| 1 | 7.62 ± 2.17 | 7.56 ± 2.42 |
| 2 | 8.10 ± 2.01 | 8.78 ± 2.57 |
| 4 | 7.98 ± 2.10 | 9.46 ± 3.39 |
| 6 | 7.98 ± 1.91 | 8.69 ± 3.22 |
| 8 | 8.95 ± 1.99 | 9.87 ± 3.05 |
| 12 | 8.49 ± 2.07 | 10.06 ± 3.45 |
| 16 | 10.27 ± 2.24 | 10.72 ± 3.45 |
| 22 | 11.83 ± 2.97 | 11.98 ± 3.60 |
| 26 | 10.03 ± 2.32 | 11.66 ± 3.86 |
| 30 | 11.31 ± 3.53 | 11.78 ± 4.14 |
| 36 | 11.64 ± 3.34 | 12.44 ± 3.59 |
| 48 | 14.36 ± 3.74 | 13.77 ± 4.12 |
| 72 | 14.99 ± 3.99 | 14.18 ± 3.73 |
| 96 | 14.94 ± 3.64 | 13.91 ± 4.37 |
| 120 | 14.21 ± 3.65 | 13.30 ± 3.46 |
| 144 | 12.50 ± 2.83 | 12.08 ± 3.95 |

**Table 3**

| LH serum level (mU/ml) following i.m and s.c injection of 5.000 IU HCG | | |
|---|---|---|
| hours after injection | i.m. | s.c. |
| 0 | 4.00 ± 1.79 | 4.13 ± 1.17 |
| 1 | 3.70 ± 1.71 | 3.74 ± 0.99 |
| 2 | 3.34 ± 1.40 | 3.59 ± 1.26 |
| 4 | 3.39 ± 1.47 | 3.27 ± 1.14 |
| 6 | 3.01 ± 1.90 | 3.00 ± 0.98 |
| 8 | 3.42 ± 2.12 | 3.49 ± 1.21 |
| 12 | 2.37 ± 1.16 | 3.16 ± 1.21 |
| 16 | 2.49 ± 1.13 | 2.63 ± 1.15 |
| 22 | 2.01 ± 0.64 | 1.77 ± 0.83 |
| 26 | 1.54 ± 0.86 | 1.42 ± 0.39 |
| 30 | 1.79 ± 0.77 | 1.46 ± 0.73 |
| 36 | 1.56 ± 0.95 | 1.56 ± 0.59 |
| 48 | 1.41 ± 0.79 | 1.58 ± 0.79 |
| 72 | 1.53 ± 1.31 | 1.60 ± 0.94 |
| 96 | 1.71 ± 1.39 | 2.01 ± 2.21 |
| 120 | 1.05 ± 0.98 | 1.65 ± 2.17 |
| 144 | 0.91 ± 1.07 | 1.11 ± 1.01 |

**Table 4**

| FSH serum level (mU/ml) following i.m and s.c injection of 5.000 IU HCG | | |
|---|---|---|
| hours after injection | i.m. | s.c. |
| 0 | 4.03 ± 1.82 | 3.63 ± 2.61 |
| 4 | 3.79 ± 1.78 | 3.14 ± 2.33 |
| 12 | 3.43 ± 1.40 | 2.91 ± 2.02 |
| 22 | 2.99 ± 1.31 | 2.46 ± 1.82 |
| 48 | 2.02 ± 1.05 | 1.89 ± 1.54 |
| 72 | 1.69 ± 0.97 | 1.42 ± 1.47 |
| 96 | 1.62 ± 0.77 | 1.58 ± 1.83 |
| 120 | 1.21 ± 0.48 | 1.30 ± 1.64 |
| 144 | 1.02 ± 0.59 | 1.01 ± 1.51 |

The comments which follow further illustrate the data provided in the Tables.

### HCG serum concentration (Table 1)

In both groups the beta-HCG serum concentration prior to injection was at the lower detection limit (group 1: 0.41 ± 0.28; group 2: 0.26 ± 0.27 mU/ml).
Following i.m. injection the highest serum level of 406.97 ± 60.19 mU/ml was found after 6 hours. The half-life was determined as 33.6 hours. Following s.c. injection the highest value of 187.47 ± 55.9 mU/ml was reached after 16 hours, with the half-life extended to 36.8 hours.

### Testosterone serum level (Table 2)

Prior to injection the testosterone values were similar in both groups (group 1: 7.94 ± 2.31, group 2: 7.74 ± 2.70 ng/ml).
The test persons of group 1 reached their highest serum concentration (14.99 ± 3.99 ng/ml) after 72 hours. The increase factor (testosterone maximum/testosterone basal) was 1.89.
Test group 2 reached their highest testosterone value (14.18 ± 3.73 ng/ml) also after 72 hours, comparable with group 1. The increase factor was 1.83.

### LH serum level (Table 3)

Prior to injection the LH serum level was approximatively equal in both groups (group 1: 4.00 ± 1.79 mU/ml, group 2: 4.13 ± 1.17 mU/ml). After administration of HCG these values decreased, showing their lowest values after 144 hours in each case: group 1 with 0.91 ± 1.07 mU/ml and group 2 with 1.11 ± 1.01 mU/ml.

### FSH serum level (Table 4)

The FSH serum level behaved in a similar manner: basal value in group 1: 4.03 ± 1.82 mU/ml and in group 2: 3.63 ± 2.61 mU/ml. After 144 hours the value had dropped to its lowest level to 1.02 ± 0.59 mU/ml in group 1, and to 1.01 ± 1.51 mU/ml in group 2..

No adverse effects were observed following s.c. injection of HCG. Side effects on the site of injection were not observed.

## Claims

1. Use of Human Chorionic Gonadotropin for the manufacture of a non-depot medicament for use in the treatment by subcutaneous administration of infertility or male sexual discorders.

## Patentansprüche

1. Verwendung von menschlichem Choriongonadotropin zur Herstellung eines Nicht-Depot-Medikaments zur Verwendung bei der Behandlung von Infertilität oder männlichen Sexualstörungen durch subkutane Verabreichung.

## Revendications

1. Utilisation de gonadotrophine chorionique humaine pour la fabrication d'un médicament pour traitement ambulatoire utilisable dans le traitement par administration sous-cutanée de l'infertilité ou de troubles sexuels masculins.
